# EUROPEAN PATENT SPECIFICATION

(11) **EP 2 773 329 B1**
(45) Date of publication and mention of the grant of the patent: **13.01.2016**
(21) Application number: 11788087.2
(22) Date of filing: 04.11.2011
(51) Int. Cl.: A61K 9/14, A61K 31/785, A61K 9/00

(54) **PHARMACEUTICAL COMPOSITIONS COMPRISING SEVELAMER**
PHARMAZEUTISCHE ZUSAMMENSETZUNGEN MIT SEVELAMER
COMPOSITIONS PHARMACEUTIQUES COMPRENANT DU SÉVÉLAMER

(43) Date of publication of application: 10.09.2014
(73) Proprietor: Synthon BV, 6545 CM Nijmegen (NL)
(72) Inventor: BAKKER-HOLMDAHL, Lisa, 6545 CM Nijmegen (NL)
(74) Representative: Mendivil Gil, Maria Dolores
(86) International application number: PCT/EP2011/069438
(87) International publication number: WO 2013/064193

(56) References cited:
- WO-A2-2007/035313
- WO-A2-2010/086881
- US-A1- 2009 155 368
- R. RADKE: "FORMULATION AND EVALUATION OF ORODISPERSIBLE TABLETS OF BACLOFEN", INTERNATIONAL JOURNAL OF CHEMTECH RESEARCH, vol. 1, no. 3, 1 July 2009 (2009-07-01), pages 517-521, XP55028664, ISSN: 0974-4290

## Description

The present invention relates to oral powder pharmaceutical compositions comprising the active pharmaceutical ingredient sevelamer carbonate.

### BACKGROUND OF THE INVENTION

Sevelamer is a non-absorbed phosphate binding polymer used in the treatment for the control of serum phosphorus in patients with chronic kidney disease (CKD). It is a product of a crosslinking of polyallyl amine with epichlorohydrine and its chemical structure is as follows:

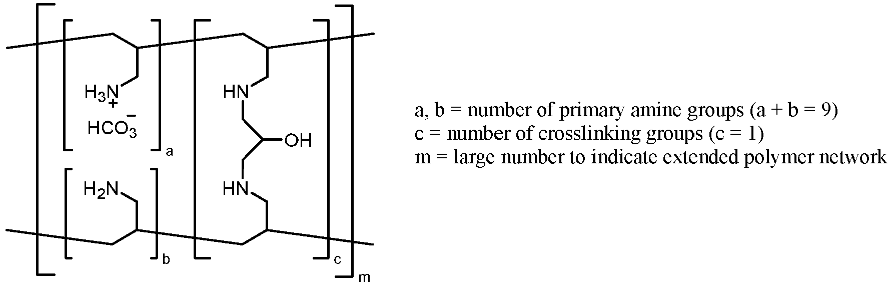

The compound contains multiple amines that become partially protonated in the intestine and interact with phosphate ions through ionic and hydrogen bonding. By binding phosphate in the gastrointestinal tract facilitating phosphorus excretion in feces, sevelamer lowers the plasma phosphorus concentration. The use of sevelamer and its pharmaceutical compositions, and processes for its preparation are disclosed in EP 0716606, EP 0831857, EP 1133989 and EP 1676581.

Sevelamer may form acid addition salts and is currently marketed either as sevelamer carbonate (Renvela®) and/or as sevelamer hydrochloride (Renagel®).

Apart from a standard tablet form, sevelamer carbonate is marketed also as a "powder for oral suspension". This dosage form comprises a foil lined, heat sealed packet - a sachet - filled with a powder comprising 1.6 or 2.4 g of sevelamer carbonate (calculated on anhydrous basis). Inactive ingredients are natural and artificial citrus flavour, propylene glycol alginate, sodium chloride, sucralose and yellow ferric oxide. The powder is administered by a patient by opening the sachet, suspending its content into a predetermined amount of water and drinking the formed suspension. Normally, the powder is administered three times a day with meals; based on clinical studies, the average prescribed daily dose of sevelamer carbonate is 7.2 g, but quantities up to 14 grams per day are allowed by medicinal authorities.

Apparently, the above sachet dosage form has been disclosed in the patent application WO 2007/035313.

Oral administration of a powder comprising relatively high amount of sevelamer carbonate is accompanied with some disadvantages affecting patient's comfort. First of all, sevelamer carbonate has a very gritty feeling in the mouth. Although this has been apparently addressed by the originator of the marketed product by adding propylene glycol alginate into the composition, the results are not optimal. Additionally, emptying the sachet during preparation of the oral suspension is accompanied with formation of a dust. This feature should also be improved.

Thus, the organoleptic properties of powder compositions for oral administration of sevelamer carbonate exhibit some disadvantages that affect patient's comfort and an improvement in this respect is quite desirable. No prior art document has however addressed the problem of improving organoleptic properties of pharmaceutical powder composition comprising sevelamer carbonate.

Thus, there exists a need of a certain improvement in the art. In particular, there exists an objective need of improvement of organoleptic properties of the powdered medicinal product comprising sevelamer carbonate , particularly in terms of taste and grittiness; such improvement, albeit not affecting the activity and safety of the drug, will prevent potential patient's discomfort when using the medical product.

### SUMMARY OF THE INVENTION

The invention relates to means of improvement of organoleptic properties of sevelamer carbonate.

In a first aspect, the invention relates to a use of a mixture of microcrystalline cellulose and sodium carboxymethyl cellulose as an agent for improvement of organoleptic properties of a pharmaceutical powder composition comprising sevelamer carbonate.

In a second aspect, the invention relates to a powder composition for oral suspension of sevelamer carbonate, such composition comprising 100 weight parts of sevelamer carbonate and 4-10 weight parts of a mixture of microcrystalline cellulose and sodium carboxymethyl cellulose, wherein preferably the relative amount of the sodium carboxymethyl cellulose in the mixture is from 10 to 12 weight %.

In a third aspect, the invention relates to a powder composition for oral suspension of sevelamer carbonate comprising a population of particles of sevelamer carbonate, which meets at least two of these three particle size distribution criteria: d₁₀ at least 30 micrometers, d₅₀ at least 60 micrometers and d₉₀ at least 120 and less than 180 micrometers.

### DETAILED DESCRIPTION OF THE INVENTION

The present invention relates to powder compositions for oral administration of sevelamer carbonate; it also relates to means and processes that are contributory to improvement of physical and organoleptic properties, in particular of taste and grittiness, of sevelamer carbonate, especially in powder pharmaceutical compositions comprising sevelamer carbonate.

The "organoleptic properties", as commonly used in the art, relate within this invention to properties that affect the human sensory organs: odour, taste, sight, feel etc. These properties are mostly not measurable by objective methods; although some organoleptic tests exist, the final outcome usually depends on an expert's organoleptic determination. For pharmaceuticals and foods, important organoleptic properties are the taste, texture, and astringency (perceived in the mouth) and aroma (perceived in the nose).

In a subjective inspection, sevelamer carbonate inherently gives a very gritty feeling in the mouth. Although the marketed powder composition of sevelamer carbonate comprises excipients that may aid to mask this feeling - particularly propylene glycol alginate - such feeling still persists in certain aspects.

The second occasion, at which human's senses are affected by sevelamer carbonate powder composition, is the moment of opening the sachet and emptying it. At this case, a certain portion of dust may evolve and such dust may exhibit an unpleasant "chemical" smell. The degree of evolution of dust may be associated also with physical properties of the powder, particularly with its flowability.

A lot of excipients have been studied with the aim to improve overall organoleptic properties of sevelamer compositions perceived in the mouth. As no objective test may be applied for the purpose, a panel of adult volunteers of both sexes and of different age has been used to evaluate the organoleptic properties subjectively and an average result was then formulated.

After thorough screening of excipients, it was found out that a mixture of microcrystalline cellulose with sodium carboxymethyl cellulose, typically comprising 10-12 weight % of sodium carboxymethyl cellulose in the mixture, has superior properties in masking the gritty feeling of sevelamer carbonate. This mixed excipient, typically as marketed under brand name Avicel CL-611 (which typically has a sodium content of about 1.2%), is generally known in foodstuff industry as a stabilizer and thickener of suspensions because of its swelling properties. However, the present invention deals with a novel use of this excipient: the use of a mixture of microcrystalline cellulose and sodium carboxymethyl cellulose for improving organoleptic properties, in particular those of sevelamer carbonate and in more particular those of powder compositions comprising sevelamer carbonate. The improvement is achieved by blending this excipient with sevelamer carbonate in a predetermined ratio, which will be discussed below. Quite surprisingly, this excipient may not only replace the propylene glycol alginate excipient used in the marketed product as a suspension thickener, but it is superior over such excipient in terms of masking the unpleasant mouthfeel of sevelamer carbonate.

The amount of the mixed microcrystalline cellulose/ sodium carboxymethyl cellulose (MCC/CMC) excipient has been tested in terms of its taste masking properties and dispersability and it was found out that optimum weight amount of this excipient is, relatively to the weight of sevelamer carbonate, from 1:0.04 to 1:0.10 (sevelamer carbonate: MCC/CMC). In other words, the powder composition for oral suspension of sevelamer carbonate of the invention advantageously comprises from 4 to 10 weight parts of the above MCC/CMC excipient per 100 weight parts of the sevelamer carbonate. Compositions with the relative amount of the MCC/CMC excipient higher than 1:0.10 form quite viscous suspensions in water and thus are less acceptable for making suspension for oral administration; compositions with the relative amount of the MCC/CMC excipient lower than 1:0.04 do not adequately mask the gritty feeling of the sevelamer carbonate. Based on the results, it further appears that the ratio sevelamer: MCC/CMC 1:0.06 (i.e. 6 weight parts of MCC/CMC excipient mixed with 100 weight parts of sevelamer carbonate) provides optimum results: the palatability is acceptable, the suspension is formed fast and only slight sedimentation takes place. In an optimum way, a composition comprising 2.4 g of sevelamer carbonate is suspended in 60 ml of water.

The problem of the "chemical smell" of sevelamer carbonate composition, which may appear during opening and emptying of some sachet containers, was studied thoroughly as well. Finally it was found out that it is the particle size distribution of sevelamer carbonate material, the proper selection of which is crucial for solving the problem of smell, whenever it might appear. Thus, in accordance with the present invention, the powder composition for oral suspension of sevelamer carbonate advantageously comprises a population of particles of sevelamer carbonate, which meets at least two of these three particle size distribution criteria: d₁₀ at least 30 micrometers, d₅₀ at least 60 micrometers and d₉₀ at least 120 and less than 180 micrometers. This means that, cumulatively, 10% of the total population of the sevelamer carbonate particles useful for making the compositions of the present invention has their maximum average particle size diameter of at least 30 micrometers, and/or 50% of the population has their maximum average particle size diameter of at least 60 micrometers and/or 90% of the population has their maximum average particle size diameter between 120 and 180 micrometers, wherein at least two of these three conditions are met.

The sevelamer carbonate particles fulfilling or not fulfilling the above particle size distribution criteria differ in various aspects, which clearly favour those within the chosen particle size distribution profile. For illustration, an example of comparison of physical properties of particles meeting and not meeting the above criteria (in further also "larger particles" and "smaller particles") is given below:

| | Batch A / not meeting (d₁₀=5µm,d₅₀=40µm,d₉₀= 108µm) | Batch B / meeting (d₁₀=40µm,d₅₀=78µm,d₉₀= 135µm) |
|---|---|---|
| Bulk density (g/ml) | 0.56 | 0.56 |
| Tapped density (g/ml) | 0.77 | 0.70 |
| Carr Index (%) | 27.9 | 20.0 |
| Haussner index | 1.39 | 1.24 |
| Ø25 mm, flow (g/s) | 15.6¹ | 32.3 |
| Angle of repose (°) | 41.5 | 40.9 |
| Ø15 mm, flow (g/s) | 4.9² | 15 |
| Angle of repose (°) | 39.3 | 41.9 |
| Ø10 mm, flow (g/s) | 1.1³ | 4.3² |
| Angle of repose (°) | 35.0 | 42.6 |

| | | |
|---|---|---|
| ¹ agitation speed 1 necessary to obtain flow ² agitation speed 2 necessary to obtain flow 3 agitation speed 4 necessary to obtain flow | | |

The Carr's index and the Haussner index was much lower for the Batch B with larger particles (20%/1.24), compared to 27%/1.39 for the Batch A with smaller particles. Using larger particles gives a better flowability. This is also clearly seen in the test of flowability through funnels with different diameters. The blend of the large particles flows much faster through all funnels and in the two largest funnels no agitation speed is needed to achieve flowability. Using the smaller particles, agitation was needed in all funnels; in the smallest funnel harder agitation was needed than in the larger funnel.

Compositions similar to the marketed compositions with various particle sizes of sevelamer carbonates were studied with respect to organoleptic properties. Using the larger particles, there is no dust when emptying the sachet into a glass of water neither when stirring to disperse the powder. The degree of chemical smell may accordingly be lower than in case of smaller particles.

In an important technological aspect, no dustiness should occur during production process. If dustiness occurs there may be problems with sealing the sachet properly. From this point of view it is absolutely necessary to use sevelamer carbonate with the above characteristics corresponding to "large particles". Filling the sachet is a critical step during manufacturing.

The above results clearly demonstrate the superiority of particles of the particle size distribution of the invention.

Thus, preferably, a useful powder composition for oral suspension of sevelamer carbonate comprises:
= 100 weight parts of a population of particles of sevelamer carbonate, which meets at least two of these three particle size distribution criteria: d₁₀ at least 30 micrometers, d₅₀ at least 60 micrometers and d₉₀ at least 120 and less than 180 micrometers;
= 4-10 weight parts of a mixture of microcrystalline cellulose and sodium carboxymethyl cellulose, wherein preferably the relative amount of the sodium carboxymethyl cellulose in the mixture is from 10 to 12 weight %.

Additionally, the composition may also comprise auxiliary excipients such as a suitable flavouring agent, a sweetener, a colourant etc., as known in the art.

The compositions of the present invention may be made by a simple process comprising blending the population of particles of sevelamer carbonate, preferably of a particle size distribution as specified above, with the suitable amount of the mixed excipient comprising microcrystalline cellulose admixed with 10-12% of sodium carboxymethyl cellulose, and with additional auxiliary excipients. The blend is weighed in portions comprising therapeutic amount of sevelamer carbonate and filled into sachets that are finally sealed.

The final sachet dosage form advantageously comprises the composition of the present invention comprising from 0.5 to 3.0 g of sevelamer carbonate, calculated on anhydrous basis, typically 1.6 or 2.4 g.

The invention will be further described with reference to the following non-limiting examples.

### EXAMPLES

### Example 1

A population of sevelamer carbonate particles (d₁₀=40 µm,d₅₀=78 µm,d₉₀=135 µm) was blended with Avicel CL-611, flavour and sucralose in Turbula blender, for 20 min. Blend corresponding to 2.4 g of anhydrous sevelamer carbonate was filled into sachets.

Compositions:

| | Amount (mg/sachet) | | | | | |
|---|---|---|---|---|---|---|
| Sevelamer carbonate | 2508 | 2508 | 2508 | 2508 | 2508 | 2508 |
| Avicel CL-611 | 150 | 150 | 150 | 150 | 150 | 150 |
| Sucralose | 9.0 | 9.0 | 9.0 | 9.0 | 9.0 | 9.0 |
| Lemon juice flavour | 115.0 | 115.0 | 86.3 | 115.0 | 86.3 | 86.3 |
| Orange flavour | 58.0 | 29.0 | 29.0 | 0.0 | 0.0 | 86.3 |
| Total weight | 2840.6 | 2811.0 | 2782.3 | 2782.0 | 2753.3 | 2839.6 |

The invention having been described it will be obvious that the same may be varied in many ways and all such modifications are contemplated as being within the scope of the invention as defined by the following claims.

## Claims

1. The use of a mixture of microcrystalline cellulose and sodium carboxymethyl cellulose as an agent for improvement of organoleptic properties of a pharmaceutical powder composition comprising sevelamer carbonate.

2. The use according to claim 1, wherein the mixture comprises 10-12 weight % of sodium carboxymethyl cellulose.

3. The use according to any of claims 1-2, wherein the composition is obtained by blending of microcrystalline cellulose and sodium carboxymethyl cellulose with sevelamer carbonate particles.

4. The use according to any of claims 1-3, wherein the ratio between sevelamer carbonate and the mixture in the blend is from 1 :0.04 to 1 :0.10 ( w/w).

5. The use according to any of claims 1-4, wherein the ratio between sevelamer carbonate and the mixture in the blend is 1 :0.06 (w/w).

6. The use according to any of claims 1-5, wherein the sevelamer carbonate particles meet at least two of these three particle size distribution criteria: d₁₀ at least 30 micrometers, d₅₀ at least 60 micrometers and d₉₀ at least 120 and less than 180 micrometers.

7. A powder composition for oral suspension of sevelamer carbonate comprising 100 weight parts of sevelamer carbonate and 4-10 weight parts of a mixture of microcrystalline cellulose and sodium carboxymethyl cellulose.

8. A powder composition for oral suspension according to claim 7, wherein the relative amount of the sodium carboxymethyl cellulose in the mixture is from 10 to 12 weight %.

9. The composition according to any of claims 7-8, wherein the population of particles of sevelamer carbonate in the composition meets at least two of these three particle size distribution criteria: d₁₀ at least 30 micrometers, d₅₀ at least 60 micrometers and d₉₀ at least 120 and less than 180 micrometers.

10. A sachet dosage form comprising the composition according to any of claims 7-9, wherein such dosage form comprises from 0.5 to 3.0 g of sevelamer carbonate, calculated on anhydrous basis.

11. A sachet dosage form comprising the composition according to any of claims 7-10, wherein such dosage form comprises 1.6 g of sevelamer carbonate, calculated on anhydrous basis.

12. A sachet dosage form comprising the composition according to any of claims 7-11, wherein such dosage form comprises 2.4 g of sevelamer carbonate, calculated on anhydrous basis.

## Patentansprüche

1. Verwendung einer Mischung von mikrokristalliner Cellulose und Natrium-Carboxymethyl-Cellulose als einen Wirkstoff für die Verbesserung organoleptischer Eigenschaften einer pharmazeutischen Pulver-Zusammensetzung umfassend Sevelamer-Carbonat.

2. Verwendung nach Anspruch 1, wobei die Mischung 10-12 Gewichts-% Natrium-Carboxymethyl-Cellulose umfasst.

3. Verwendung nach einem beliebigen der Ansprüche 1-2, wobei die Zusammensetzung durch Mischen von mikrokristalliner Cellulose und Natrium-Carboxymethyl-Cellulose mit Sevelamer-Carbonat-Partikeln erhalten ist.

4. Verwendung nach einem beliebigen der Ansprüche 1-3, wobei das Verhältnis zwischen Sevelamer-Carbonat und der Mischung in dem Blend 1:0,04 bis 1:0,10 (Gew./Gew.) beträgt.

5. Verwendung nach einem beliebigen der Ansprüche 1-4, wobei das Verhältnis zwischen Sevelamer-Carbonat und der Mischung in dem Blend 1:0,06 (Gew./Gew.) beträgt.

6. Verwendung nach einem beliebigen der Ansprüche 1-5, wobei die Sevelamer-Carbonat-Partikel mindestens zwei der folgenden drei Partikelgrößenverteilungskriterien erfüllen: d₁₀ mindestens 30 Mikrometer, d₅₀ mindestens 60 Mikrometer und d₉₀ mindestens 120 und weniger als 180 Mikrometer.

7. Pulver-Zusammensetzung für orale Suspension von Sevelamer-Carbonat umfassend 100 Gewichtsteile Sevelamer-Carbonat und 4-10 Gewichtsteile einer Mischung von mikrokristalliner Cellulose und Natrium-Carboxymethyl-Cellulose.

8. Pulver-Zusammensetzung für orale Suspension nach Anspruch 7, wobei die relative Menge von Natrium-Carboxymethyl-Cellulose in der Mischung von 10 bis 12 Gewichts-% beträgt.

9. Zusammensetzung nach einem beliebigen der Ansprüche 7-8, wobei die Bevölkerung (Population) von Partikeln von Sevelamer-Carbonat in der Zusammensetzung mindestens zwei der folgenden drei Partikelgrößenverteilungskriterien erfüllt: d₁₀ mindestens 30 Mikrometer, d₅₀ mindestens 60 Mikrometer und d₉₀ mindestens 120 und weniger als 180 Mikrometer.

10. Dosierbeutelform umfassend die Zusammensetzung gemäß einem beliebigen der Ansprüche 7-9, wobei die Dosierungsform von 0,5 bis 3,0 g Sevelamer-Carbonat, berechnet auf die Trockensubstanz, umfasst.

11. Dosierbeutelform umfassend die Zusammensetzung nach einem beliebigen der Ansprüche 7-10, wobei die Dosierungsform 1,6 g Sevelamer-Carbonat, berechnet auf die Trockensubstanz, umfasst.

12. Dosierbeutelform umfassend die Zusammensetzung nach einem beliebigen der Ansprüche 7-11, wobei die Dosierungsform 2,4 g Sevelamer-Carbonat, berechnet auf die Trockensubstanz, umfasst.

## Revendications

1. L'utilisation d'un mélange de cellulose microcristalline et de carboxyméthylcellulose de sodium en tant qu'agent d'amélioration des propriétés organoleptiques d'une composition de poudre pharmaceutique comprenant du carbonate de sévélamer.

2. L'utilisation selon la revendication 1, dans laquelle le mélange comprend 10 à 12% en poids de carboxyméthylcellulose de sodium.

3. L'utilisation selon une quelconque des revendications 1-2, dans laquelle la composition est obtenue par mélange de cellulose microcristalline et de carboxyméthylcellulose de sodium avec des particules de carbonate de sévélamer.

4. L'utilisation selon une quelconque des revendications 1-3, dans laquelle le rapport entre le carbonate de sévélamer et le mélange dans le mélange est compris entre 1/ 0,04 et 1/ 0,10 (en poids/poids),

5. L'utilisation selon une quelconque des revendications 1-4, dans laquelle le rapport entre le carbonate de sévélamer et le mélange dans le mélange est de 1/0,06 (en poids/poids).

6. L'utilisation selon une quelconque des revendications 1 à 5, dans laquelle les particules de carbonate de sévélamer répondent à au moins deux des trois critères de répartition des dimensions de particules suivants:
- d₁₀ d'au moins 30 micromètres,
- d₅₀ d'au moins 60 micromètres et
- d₉₀ d'au moins 120 et inférieur à 180 micromètres.

7. Une composition de poudre pour suspension orale de carbonate de sévélamer comprenant 100 parties en poids de carbonate de sévélamer et 4-10 parties en poids d'un mélange de cellulose microcristalline et de carboxyméthylcellulose de sodium.

8. Une composition de poudre pour suspension orale selon la revendication 7, dans laquelle la quantité relative de carboxyméthylcellulose de sodium dans le mélange est de 10 à 12% en poids.

9. La composition selon une quelconque des revendications 7-8, dans laquelle la population de particules de carbonate de sévélamer dans la composition répond à au moins deux des trois critères de répartition de dimensions suivants:
- d₁₀ d'au moins 30 micromètres,
- d₅₀ d'au moins 60 micromètres et
- d₉₀ d'au moins 120 et inférieur à 180 micromètres.

10. Une forme de dosage de sachet comprenant la composition selon une quelconque des revendications 7-9, dans laquelle la forme posologique comprend de 0,5 à 3,0 g de carbonate de sévélamer, calculés sur une base anhydre.

11. Une forme de dosage de sachet comprenant la composition selon une quelconque des revendications 7-10, dans laquelle une telle forme posologique comprend 1,6 g de carbonate de sévélamer, calculés sur une base anhydre.

12. Une forme de dosage de sachet comprenant la composition selon une quelconque des revendications 7-11, dans laquelle une telle forme posologique comprend 2,4 g de carbonate de sévélamer, calculés sur une base anhydre.
